(19) Europäisches Patentamt

European Patent Office

Office européen des brevets

(11) **EP 3 821 793 A1**

(12) **EUROPEAN PATENT APPLICATION**

(43) Date of publication:
19.05.2021 Bulletin 2021/20

(21) Application number: 19208425.9

(22) Date of filing: 12.11.2019

(51) Int Cl.:
*A61B 5/00* (2006.01)    *G04G 13/02* (2006.01)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Koninklijke Philips N.V.
5656 AG Eindhoven (NL)

(72) Inventors:
• TSONEVA, Tsvetomira Kirova
5656 AE Eindhoven (NL)
• GARCIA MOLINA, Gary
5656 AE Eindhoven (NL)
• CHEN, Xia
5656 AE Eindhoven (NL)

(74) Representative: Philips Intellectual Property &
Standards
High Tech Campus 5
5656 AE Eindhoven (NL)

(54) **A METHOD FOR DETERMINING THE RISK OF A USER WAKING UP IN AN UNDESIRABLE STATE**

(57) A method is provided for measuring a wake up indicator, where the wake up indicator gives the likelihood of a user waking up undesirably. It is based on the knowledge that waking up during deep NREM sleep is not desirable due to sleep inertia and on the recognition that it is also undesirable to wake up during REM sleep due to atonia. A sleep inertia estimation is determined to estimate if the user is in NREM sleep and an atonia estimation is determined to estimate if the user is in REM sleep. The wake up indicator is determined from the sleep inertia estimation and the atonia estimation. The wake up indicator thus may be used as an indicator for the time when it is suitable to wake up the user in a way which avoids arousal from a deep sleep state or from REM sleep, during which atonia may arise.

FIG. 1

**Description**

FIELD OF THE INVENTION

This invention relates to the field of determining the risk of a user waking up in an undesirable state. It relates in particular to measuring a wake up indicator.

BACKGROUND OF THE INVENTION

Many people are familiar with the feeling of grogginess just after the wake up alarm has gone off in the morning. This period of incomplete awakening, low arousal and reduced ability to perform simple tasks is known as sleep inertia and is more pronounced if awakening occurs from deep sleep. Studies have even shown that the impairment caused by sleep inertia can be compared to the effects of alcoholic intoxication and may be especially dangerous for shift workers that need to make important decisions soon after awakening.

The presence of slow wave (deep) sleep prior to waking up tends to exacerbate sleep inertia with deeper sleep being more detrimental to the feeling of alertness in the morning. The ratio between high and low frequencies in the sleeping EEG seems to be a good estimate of depth of sleep and could be a good predictor of sleep inertia.

Atonia (the complete lack of tone of somatic muscles) has been found to occur in humans during REM sleep. In humans and in many other species, it is well established that there is a cessation of muscle tone during REM sleep. Somatic motoneurons are hyperpolarized (i.e. less active) during REM sleep as compared with NREM sleep (and wakefulness). During the transition from REM to wakefulness, the somatic motoneurons depolarize but that process is not necessarily immediate which can cause a feeling of muscle paralysis when waking up from REM sleep.

The severity of sleep inertia and sleep paralysis builds up with time.

To avoid the sleep inertia effects, many intelligent alarm clocks, such as smart alarms, have appeared in recent years. Their claims revolve around the idea that sleep stages can be monitored unobtrusively with various physiological sensors (e.g. EEG, actigraphy, PPG, etc.) and the person is woken up whenever lighter sleep (stage N1 or N2) is detected. Those solutions rely on the fact that waking up from N1 or N2 reduces sleep inertia (which occurs when waking-up from N3), but disregard the risk of muscle atonia when woken up from REM sleep.

A conventional smart alarm uses a pre-defined time period (window, e.g. 30 minutes) around the user-defined desired wake up time, and makes sure that the alarm is triggered at a moment of light sleep. Typical smart alarm algorithms continuously monitor the sleep stages of the user in that window and whenever a light sleep is detected the user is woken up. To make sure the user does not transition to deep sleep again, this typically happens at the first instance of light sleep. Given that the probability of lighter sleep in the morning is rather high, this usually happens in the beginning of the window, i.e. 30 minutes before the user-defined desired wake up time. Thus, a typical complaint from users using such alarms is that they are woken up too early and are deprived from the possibility to sleep longer.

These solutions also do not take into account the disadvantages associated with waking up from REM sleep and the fact that sleep inertia is analog i.e. a continuous process rather than binary (yes/no state).

Therefore there is a need for a method for determining when to wake a user up which can take into account the negative effects of waking up during REM sleep.

SUMMARY OF THE INVENTION

The invention is defined by the claims.

According to examples in accordance with an aspect of the invention, there is provided a method for determining a wake up indicator, wherein the wake up indicator measures the risk of a user waking up in an undesirable state, the method comprising:

determining a sleep inertia estimation based on at least one physiological signal of the user for estimating if the user is in NREM sleep;
determining an atonia estimation based on at least one physiological signal of the user for estimating if the user is in REM sleep; and
using the inertia estimation and the atonia estimation to determine the wake up indicator.

The wake up indicator gives the likelihood of a user waking up undesirably. It is based on the knowledge that waking up during deep NREM sleep is not desirable due to sleep inertia. Sleep inertia is the period of incomplete awakening, low arousal and reduced ability to perform simple tasks, and is more pronounced when awakening during deep sleep. Deep sleep is generally categorized as N3 sleep. The sleep inertia estimation is a continuous estimation of sleep depth based on physiological signals, such electroencephalogram (EEG) signals.

The wake up indicator also depends on the atonia estimation. Atonia (complete lack of tone of somatic muscles) has been found to occur during REM sleep as explained above. The invention is based on the recognition that it is also undesirable to wake up during REM sleep. The atonia estimation may be based on the duration of the current REM sleep episode which can be estimated from physiological signals, such as from muscle activity and eye movement.

The wake up indicator thus may be used as an indicator for the time when it is suitable to wake up the user in a way which avoids arousal from a deep sleep state or from REM sleep, during which atonia may arise.

The wake up indicator may be determined as a weighted sum of the sleep inertia estimation and the atonia estimation. Thus, an analog measurement may be provided as the wake up indicator.

The physiological signal may be determined using an electroencephalogram, EEG, electrocardiogram ECG, photopletismogram PPG, and/or an electromyogram, EMG.

The invention also provides a wake up indicator computer program, such as a mobile application, comprising code means for implementing the method for determining the wake up indicator when said program is run on a processing system.

The invention also provides a method for actuating an alarm at an optimal time for a user, comprising:

receiving a desired time to actuate the alarm, and thus to wake up, from the user;
receiving a time window from the user, the time window defining a set of times, before the desired time, during which the alarm can be actuated;
checking the time is within the time window;
in response to the time being within the time window:

determining the wake up indicator using the method described above;
determining if the wake up indicator is within a pre-determined range; and
actuating the alarm when the wake up indicator is within the pre-determined range or when the desired time is reached.

A user sets a time at which they would like to wake up, a desired time, and a time window in which they wouldn't mind waking up, such as 30 minutes before the desired time. The time is checked to see if it is within the specified time window. If the time is within the time window, then the time is continuously checked again to see if it is the desired time to wake up. If it is, then an alarm is actuated to wake the user up. If the time is not the desired time, then the wake up indicator is determined and is checked to determine if it falls within a particular range, for example by comparison with a pre-determined threshold. If the wake up indicator is above the pre-determined threshold, then the alarm is actuated to wake the user up.

The pre-determined threshold may be adapted to the user. In other words, a personalized threshold can be determined. For example, an artificial intelligence processor can determine, through many uses of the device, which pre-determined threshold gives the most pleasant waking up experience and adapt the pre-determined threshold to suit the individual user.

The invention also provides an alarm actuation computer program, such as a mobile application, comprising code means for implementing the method for actuating an alarm when said program is run on a processing system.

The invention also provides a device for determining a wake up indicator, wherein the wake up indicator indicates the likeliness of a user undesirably waking up, comprising at least one sensor for sensing at least one physiological signals and a wake up indicator computer program for determining the wake up indicator.

The sensors can be used to determine the physiological signals, which are used by the computer program to calculate a sleep inertia estimation and an atonia estimation. These are used by the computer program to calculate the wake up indicator.

At least one of the sensors may be attached to the user, such as electrodes for an EEG, ECG, EMG or an EOG. At least one of the sensors may be independent from the user, such as a microphone or a camera. Thus various combination of sensors may be used.

The invention also provides a system for waking a user up at an optimal time, comprising:

a device for determining a wake up indicator, wherein the wake up indicator indicates the likeliness of a user undesirably waking up, comprising at least one sensor for sensing at least one physiological signals;
an alarm for waking the user up; and
an alarm actuation computer program for actuating the alarm.

The alarm may be adapted to deliver a stimulus, such as auditory, visual or somatosensory stimulation, to the user.

The alarm may adapted to deliver a stimulus having an intensity which increases gradually, such as an increasing sound intensity over time.

The system may be adapted to wake a user up from a daily sleep or it may be adapted to wake a user up from a short nap. For shift workers, being sharp and ready to perform immediately after waking up from a short sleep is crucial. The system would avoid the user from waking up during deep sleep or REM sleep, whilst allowing the user to sleep for as long as possible.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows a process used to measure a wake up indicator;
Figure 2 shows a process used to determine when to actuate an alarm;
Figure 3 shows an example hypnogram;
Figure 4 shows an alarm system in schematic form;
Figure 5 shows a first example of how the performance of the alarm system compares to a known alarm system;
Figure 6 shows a second example of how the performance of the alarm system compares to a known alarm system; and
Figure 7 shows the components of the alarm system .

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for measuring a wake up indicator, where the wake up indicator gives the likelihood of a user waking up undesirably. It is based on the knowledge that waking up during deep NREM sleep is not desirable due to sleep inertia and on the recognition that it is also undesirable to wake up during REM sleep due to atonia. A sleep inertia estimation is determined to estimate if the user is in NREM sleep and an atonia estimation is determined to estimate if the user is in REM sleep. The wake up indicator is determined from the sleep inertia estimation and the atonia estimation. The wake up indicator thus may be used as an indicator for the time when it is suitable to wake up the user (using an alarm system) in a way which avoids arousal from a deep sleep state or from REM sleep, during which atonia may arise.

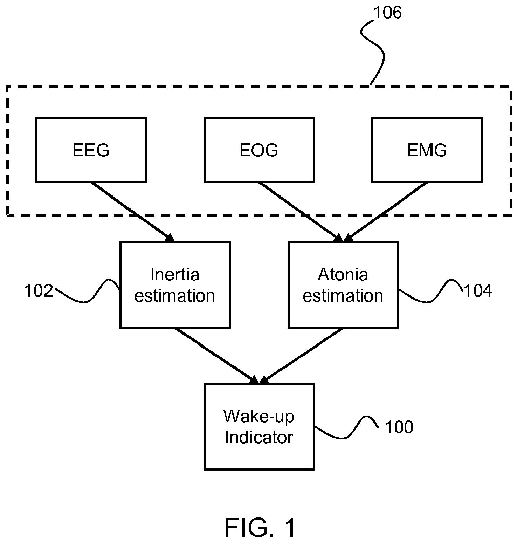

Figure 1 shows the method used to find the wake up indicator 100. The wake up indicator 100 is a continuous value assessing the chance of an undesirable wake up state (i.e. severe sleep inertia or muscle paralysis). It combines two elements, a sleep inertia estimation 102, or inertia index, and an atonia estimation 104, or atonia index. One possible way of calculating the wake up indicator is:

$$wake\_up\ indicator(t) = w_I(t)^* inertia\ index(t) + w_A(t)^* atonia\ index(t) \qquad (1)$$

This defines a weighted summation of an inertia index and an atonia index. It evolves with time, so that at any point in time, it can be determined if it is appropriate to wake up the user.

The inertia index 102 can be measured from the continuous estimation of sleep depth, based on sleep stages. Sleep stages, which occur in a cyclic manner (sleep cycles) throughout a night of sleep, can be characterized by specific patterns of physiological signals 106 and can be reliably monitored in real time during sleep.

Sleep can be monitored utilizing many different physiological signals 106 or combinations thereof, such as:

Electroencephalogram (EEG) from a sensor attached to the head;
Electro-oculogram (EOG) from a sensor attached near the eyes;
Actigraphy signals from sensors attached to the body (e.g. wrist or leg) or placed on the bed;

Photoplethysmogram signals (PPG) from an optical sensor;
Electrocardiogram (ECG) signals from electrodes attached to the body;
Respiratory effort signal from breathing sensors (belt, radar, or nasal cannula);
Body position signals using pressure sensor(s) in the mattress or a camera;
Body movement signals from radar sensors or a camera;
Audio signals characterizing breathing sounds or snoring capture through a microphone; and
Brain activity using a optical sensor (functional near-infrared fNIR).

Different sensors can be used to give an indication of sleep stages. Thus even if EEG sensors, which are primarily used for detecting sleep stages, fall off, or otherwise become non-functioning, others sensors which are present may be used to continue to determine the sleep stage.

Commonly, EEG signals may be measured and/or received using one or more electrodes positioned on the head of a subject. The amplitude of these signals, as well as the specifics of the peaks, troughs, sleep spindles, k-complexes, slow waves, and/or frequency-based characteristics within these signals may be analyzed to distinguish the current sleep stage of a subject. For example, slow waves are known to be more abundant in sleep stage 3, whereas sleep spindles may be more abundant in sleep stage 2.

Different sleep stages can be identified with these different sensing approaches.

For example, four sleep stages can be identified with polysomnography (PSG). Stage N1 and N2 are the stages of light sleep, characterized by theta (4-8 Hz) oscillatory brain activity, and sleep spindles and K-complexes respectively. Stage N3 is the stage of deep sleep characterized by slow-waves and delta activity (0.5-4 Hz). REM sleep typically occurs after around 90 minutes of sleep onset and is characterized by increased eye movement, hearth rate, and respiration. During sleep, short wake periods can also occur, this state is referred to as W.

It is also possible to provide stimuli to a subject and to use their response to provide an indication or validation of a sleep stage. This approach is for example disclosed in WO 2014/118693. In this example, stimuli may include visual stimuli, auditory stimuli, tactile stimuli, olfactory stimuli, electromagnetic stimuli, somatosensory stimuli, other sensory stimuli and/or any combination and/or sequence thereof. A stimulus source may thus include one or more of a light source, a loudspeaker, an electroacoustic transducer, a vibrating component or device, a device or system configured to produce scents or electrodes.

The invention can make use of any known sensor arrangement for providing a determination of the sleep stage of a subject.

Sleep <u>depth</u> can also be derived from an electroencephalogram (EEG) power in the delta, alpha, and beta frequency bands as follows:

$$Sleep\ Depth\ (SD) = log2\left(\frac{Delta}{Beta}\right) + log2\left(\frac{Delta}{Alpha}\right) \qquad (2)$$

This ratio is a good approximation of depth of sleep because it is known that as sleep deepens the power in the lower frequency bands (such as delta band: 0.5 - 4 Hz) increases, whilst that the power in the higher frequency bands (such as alpha: 8 - 12 Hz, and beta: 15- 30 Hz) decreases. Taking the ratio between these signals reduces possible artefacts or noise in either of the signals. In practice, this ratio is easily calculated in real-time from the filtered EEG in the delta, alpha and beta bands. The running average of these signals can be computed (e.g. in a 1.5 second window) to further smooth the estimate.

A simpler version of sleep depth estimation can comprise only the ratio between the delta and the beta band, the delta and the alpha band, or any ratio between frequencies in the higher end vs. the lower end of the EEG spectrum.

The inertia estimation or inertial index 102 is obtained from the sleep stage and optionally depth of sleep information as explained above.

The atonia estimation or atonia index 104 can be measured from the duration of the current REM episode estimated as the time in seconds from the beginning of the episode. A different version of the atonia index 104 could comprise the amount of muscle activity as detected by an electromyograph (EMG) sensor or eye movements as detected by an electrooculograph (EOG) sensor.

The wake up indicator 100 is defined such that the relative importance of each of the two elements in the final value of the wake up indicator 100 is adjusted according to the two weights (i.e. $w_1$ and $w_A$). The weights can be any real number.

Sleep history can also be incorporated by extending the time window t into the past and defining weights $w_I$ and $w_A$ in the form of a vector (which is equivalent to applying a filter) or by calculating the inertia index 102 and atonia index 104 as exponentially weighed averages, such as:

$$running\ inertia\ index(t)=\ w_I *running\ inertia\ index(t\text{-}1)+(1\text{-}\ w_I)*inertia\ index(t) \qquad (3)$$

$$running\ atonia\ index(t)=\ w_A *running\ atonia\ index(t\text{-}1)+(1\text{-}\ w_A)*atonia\ index(t) \qquad (4)$$

This approach creates a running index, which takes account of the immediately preceding running index value and the current instantaneous value.

Then the wake up indicator 100 is calculated as a simple summation:

$$wake\_up\ indicator(t)=\ running\ inertia\ index(t)+running\ atonia\ index(t) \qquad (5)$$

Both implementations (applying a filter or calculating exponentially weighted averages) are essentially similar and the adjustment of weights allow to define the amount of time in the past we want to consider for the calculation of the wake up indicator 100. Such implementation of the wake up indicator 100 will provide a smoother behavior of the system by filtering out noise and disregarding very short transitions between stages.

In essence, the proposed algorithm can prevent waking up a user at the wrong stages (REM/N3).

In the case of alternative sleep staging classifications, the above algorithms can be applied as well. If only light and deep sleep are distinguished, then conditions regarding stages N1,N2 and W will be applied to light sleep, while conditions regarding REM or N3 will be applied to deep sleep. In the case of continuous sleep depth monitoring a simple threshold could be used to distinguish between light and deep sleep and the algorithms translated as explained above.

One application of interest for the wake up indicator is for use in an alarm.

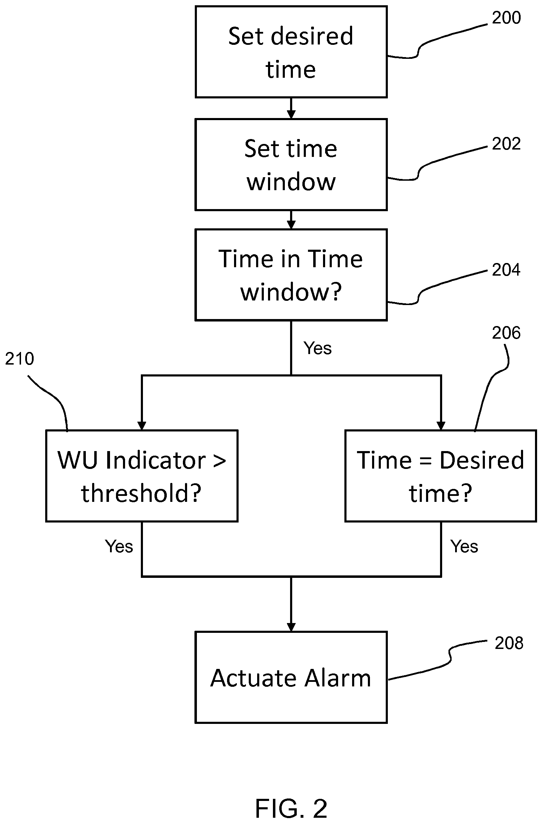

Figure 2 shows a process used to determine when to actuate an alarm based on the wake up indicator measured using the method explained above. The method reduces the gap between the user's desired wake up time and alarm-triggered wake up time based on the real-time estimation of sleep stages and an introduction of a delayed decision window.

A user sets a time (a single time value) at which they would like to wake up, named a desired time, in step 200.

The user also sets a time window in step 202 during which they wouldn't mind waking up, such as 30 minutes before the desired time.

In step 204, the time is checked to see if it is within the specified time window. If the time is within the time window, then the time is continuously checked again in step 206 to see if it has reached the desired time to wake up. If it is, then an alarm is actuated in step 208 to wake the user up.

While the time has not yet reached the desired time, the wake up indicator determined as explained above is also checked in step 210 to determine if it falls within a range, for example by comparison with a pre-determined threshold. If the wake up indicator is above the pre-determined threshold, then the alarm is actuated in step 208 to wake the user up.

The pre-determined threshold may be adapted to the user. In other words, a personalized threshold can be determined. For example, an artificial intelligence processor can determine, through many uses of the device, which pre-determined threshold gives the most pleasant waking up experience and adapt the pre-determined threshold to suit the individual user.

In essence, the proposed method increases the probability for waking up near the desired time. The method promotes a pleasant wake up experience without curtailing sleep.

The method thus continuously monitors the sleep of a user in the predefined alarm window and continuously calculates the wake up indicator. The system postpones the alarm until the value of the wake up indicator exceeds a predefined threshold or until the desired wake up time of the user has been reached. This increases the probability for pleasant wake up naturally close to (but not later than) the desired time disregarding the notion of discrete sleep stage transitions (ON/OFF switch) used in most smart alarm solutions.

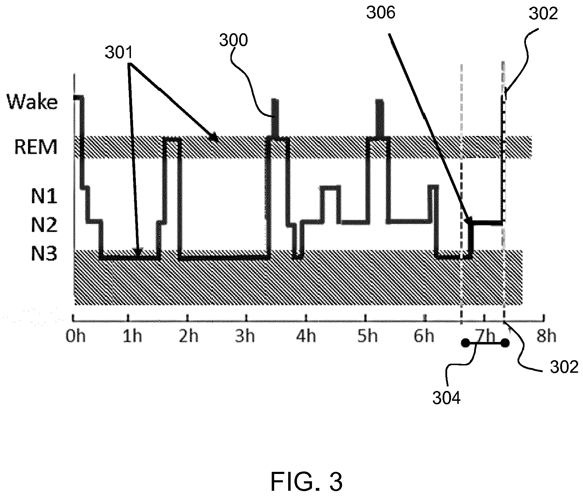

Figure 3 shows an example hypnogram. The line 300 depicts the sleep architecture (hypnogram) in a typical night of sleep. When the value falls in the REM and N3 bands 301 there is muscle paralysis (REM) and high sleep inertia (N3) if the user is woken up. In this example, the user's desired wake up time 302 is 7:15am and a 30 minute-long alarm window 304 was preset so that wakeup may occur between 6:45 am and 7:15 am.

In this example, a conventional smart alarm system will have to wake up the user at the moment they exit N3 sleep (at the time shown as 306, approximately 6:50am), thus, reducing the total sleep time of the user by 25 minutes. By monitoring the sleep stages of the user and measuring a wake up indicator depending on the detected sleep stage and atonia condition, the alarm system may only wake up the user if the value of the wake up indicator exceeds a predefined threshold or if it is the desired time 302.

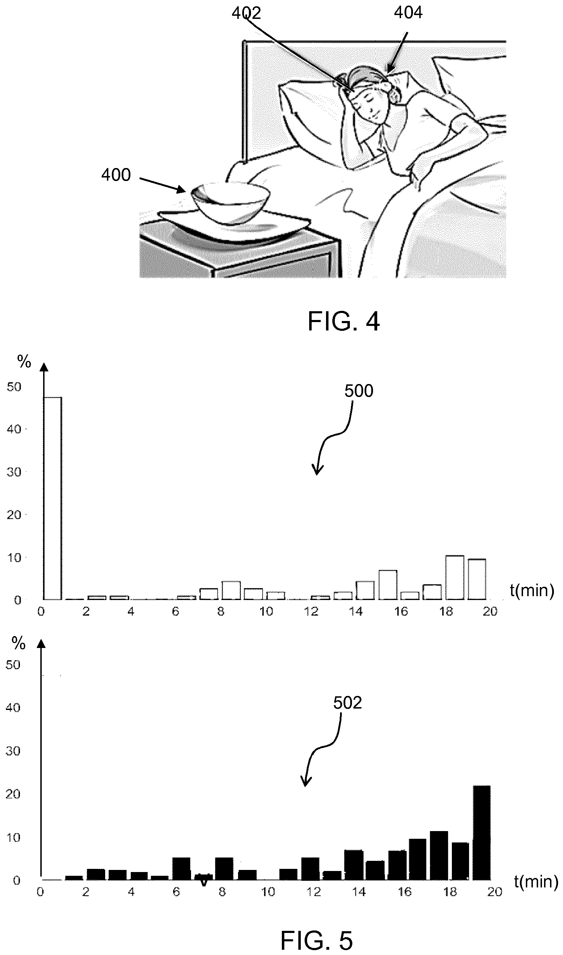

Figure 4 shows an example of an alarm system used to determine when to actuate an alarm 400 shown as a wake up light. In this example, the user wears a headset 402 during sleep. The headset 402 could have sensor electrodes which may measure EEG and EOG signals. The headset 402 could either comprise a processor which can determine the wake up indicator or send the sensor 402 information to a processor, such as in a mobile phone or in the alarm 400 or in any other remote processing unit, to determine the wake up indicator.

The processor may also determine when to actuate the alarm 400 to wake the user up. When the user is to be woken up, the alarm 400 is actuated. The alarm may also include an actuator 404 on the headset which is also turned on. The actuator can include a speaker and a vibration motor to wake the user up. An audio alarm remote from the user may of course also be used.

More generally, the alarm delivers auditory, visual, or somatosensory stimulation or any of their combinations. In the case of auditory stimulation, tones of increasing volume may be used. Gradual light intensity increase can also have a positive effect on the wake up experience. Further, slight vibrations of increasing intensity can also be used to ease the transition from sleep to wakefulness.

Figure 5 shows the results of simulations on historical sleep data. Figure 5 shows the amount of sleep time shortening, i.e. how much earlier the alarm was operated than the desired time, as a histogram.

The top plot 500 relates to the alarm described above and the bottom plot 502 relates to a standard smart alarm, which provides an alarm at the first time outside N3.

For this particular simulation the threshold was sufficiently small that N3 sleep or REM sleep were avoided (e.g. 0.01). In addition, both the running inertia index explained above and the running atonia index had equal importance in the score calculation and a running average over 3 windows of 6 seconds was applied (resulting in at most of 12 secs in an unfavorable sleep state).

The historical data is based on 115 sleep studies recorded at home. The study involved 27 subjects (17 Female/10 Male; $37.2 \pm 7.2$ years old). The wake up window was set to 20 minutes hence the maximum sleep time shortening of 20 minutes. The sleep shortening associated with the new method (7.8 minutes on average) is substantially shorter compared to a typical smart alarm (14.5 minutes on average). In addition, in 50% of cases, no sleep shortening at all occurs when using the method.

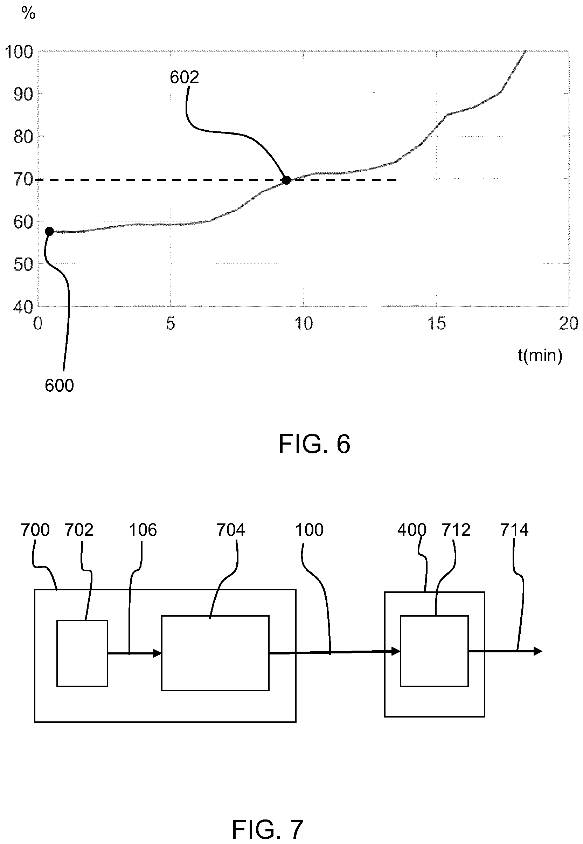

Figure 6 shows a graph of cumulative frequency versus minutes before wake up time for a second dataset (26 subjects; 16 Female /10 Male; $33.6 \pm 8.4$ years old; 2 nights/subject). The percent of cases where the user wakes up from light sleep when using the disclosed method was estimated.

As shown in Figure 6, in 57% of cases (point 600) the method will promote fresh wakeup and in 70% of cases, it will promote fresh wakeup within 10 minutes of the desired wakeup time (point 602).

All of the above embodiments are applicable both after a main daily sleep session or a short nap. In the case of a nap, e.g. for shift workers, being sharp and ready to perform immediately after wake up is crucial. In this embodiment the duration of the nap is scheduled according to the time available and deep sleep is avoided by the means described above.

The skilled person would be readily capable of developing a processor for carrying out any herein described method. Thus, each step of a flow chart may represent a different action performed by a processor, and may be performed by a respective module of the processing processor.

Figure 7 shows an alarm system which combines a device 700 for determining a wake up indicator 100 and an alarm 400. The device 700 comprises at least one sensor 702 for sensing at least one physiological signal 106 and a processor 704 which operates a wake up indicator computer program for determining the wake up indicator 100.

The wake up indictor 100 is provide to the alarm 400, for waking a user up at an optimal time. The alarm comprises a processor 712 which operates an alarm actuation computer program for actuating the alarm, shown an alarm signal 714.

The device 700 and alarm 400 may be separate systems or they may form a combined system.

The processing may be carried out locally or remotely. It may for example be carried by a mobile telephone which communicates with the sensors and with the alarm.

As discussed above, the system makes use of processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

**Claims**

1. A method for determining a wake up indicator (100), wherein the wake up indicator measures the risk of a user waking up in an undesirable state, the method comprising:

   (102) determining a sleep inertia estimation based on at least one physiological signal (106) of the user for estimating if the user is in NREM sleep;
   (104) determining an atonia estimation based on at least one physiological signal (106) of the user for estimating if the user is in REM sleep; and

   using the inertia estimation (102) and the atonia estimation (104) to determine the wake up indicator (100).

2. A method as claimed in claim 1, comprising determining the wake up indicator (100) as a weighted sum of the sleep inertia estimation (102) and the atonia estimation (104).

3. A method as claimed in claim 1 or 2, comprising determining the physiological signal (106) using an electroencephalogram, EEG, electrocardiogram ECG, photopletismogram PPG, an electro-oculogram (EOG) and/or an electromyogram, EMG.

4. A wake up indicator computer program, such as a mobile application, comprising code means for implementing the method of any one of claims 1 to 3, when said program is run on a processing system (704).

5. A method for actuating an alarm at an optimal time for a user, comprising:

   (200) receiving a desired time to actuate the alarm, and thus to wake up, from the user;
   (202) receiving a time window (304) from the user, the time window defining a set of times, before the desired time, during which the alarm can be actuated;
   (204) checking the time is within the time window;

   in response to the time being within the time window:

   determining the wake up indicator using the method as claimed in any one of claims 1 to 3;
   (210) determining if the wake up indicator (100) is within a pre-determined range; and
   (208) actuating the alarm when the wake up indicator (100) is within the pre-determined range or when the desired time (302) is reached.

6. A method as claimed in claim 5, further comprising adapting the pre-determined range to the user.

7. An alarm actuation computer program, such as a mobile application, comprising code means for implementing the method of any one of claims 5 or 6 when said program is run on a processing system (712).

8. A device (700) for determining a wake up indicator (100), wherein the wake up indicator (100) measures the risk of a user undesirably waking up, comprising:

   at least one sensor (702) for sensing at least one physiological signal (106);
   a processor (704); and
   a wake up indicator computer program, as claimed in claim 4, operated by the processor for determining the wake up indicator (100).

9. A device as claimed in claim 8, wherein at least one of the sensors (702) is attached to the user, such as electrodes for an EEG, ECG, EMG or an EOG.

10. A device as claimed in claim 8 or 9, wherein at least one of the sensors is independent from the user, such as a microphone or a camera.

11. A system for waking a user up at an optimal time, comprising:

a device (700) for determining a wake up indicator (100), wherein the wake up indicator (100) measures the risk of a user undesirably waking up, comprising at least one sensor (702) for sensing at least one physiological signals (106);
an alarm (400) for waking the user up;
a processor (712); and
an alarm actuation computer program, as claimed in claim 7, operated by the processor for actuating the alarm (400).

12. A system as claimed in claim 11, wherein the alarm (400) is adapted to deliver a stimulus, such as auditory, visual or somatosensory stimulation, to the user.

13. A device as claimed in claim 12, wherein the alarm (400) is adapted to deliver a stimulus having an intensity which increases gradually, such as an increasing sound intensity over time.

14. A system as claimed in any of claims 11 to 13, wherein the system is adapted to wake a user up from a daily sleep.

15. A system as claimed in any of claims 11 to 13, wherein the system is adapted to wake a user up from a short nap.

FIG. 1

FIG. 2

FIG. 3

FIG. 4

FIG. 5

%

FIG. 6

FIG. 7

Europäisches
Patentamt

European
Patent Office

Office européen
des brevets

# EUROPEAN SEARCH REPORT

**Application Number**

EP 19 20 8425

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/151603 A1 (SHOULDICE REDMOND [IE] ET AL) 2 June 2016 (2016-06-02) * paragraph [0095] - paragraph [0098] * ----- | 1-15 | INV.<br>A61B5/00<br>G04G13/02 |
| X | US 2007/249952 A1 (RUBIN BENJAMIN [US] ET AL) 25 October 2007 (2007-10-25) * the whole document * ----- | 1-15 | |
| X | DE 42 09 336 A1 (KNUTZEN SOENKE [DE]; HELMS GERD [DE]) 30 September 1993 (1993-09-30) * the whole document * ----- | 1-15 | |
| | | | **TECHNICAL FIELDS SEARCHED (IPC)**<br><br>A61B<br>G04G |

The present search report has been drawn up for all claims

| Place of search | Date of completion of the search | Examiner |
|---|---|---|
| The Hague | 30 March 2020 | Van Dop, Erik |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 19 20 8425

This annex lists the patent family members relating to the patent documents cited in the above-mentioned European search report.
The members are as contained in the European Patent Office EDP file on
The European Patent Office is in no way liable for these particulars which are merely given for the purpose of information.

30-03-2020

| Patent document cited in search report | | Publication date | Patent family member(s) | | | Publication date |
|---|---|---|---|---|---|---|
| US 2016151603 | A1 | 02-06-2016 | AU | 2014287372 | A1 | 21-01-2016 |
| | | | CN | 105592777 | A | 18-05-2016 |
| | | | EP | 3019073 | A2 | 18-05-2016 |
| | | | JP | 2016532481 | A | 20-10-2016 |
| | | | US | 2016151603 | A1 | 02-06-2016 |
| | | | US | 2020009349 | A1 | 09-01-2020 |
| | | | WO | 2015006364 | A2 | 15-01-2015 |
| US 2007249952 | A1 | 25-10-2007 | DE | 102007009722 | A1 | 30-04-2008 |
| | | | US | D626240 | S | 26-10-2010 |
| | | | US | 2007249952 | A1 | 25-10-2007 |
| DE 4209336 | A1 | 30-09-1993 | NONE | | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014118693 A